Europäisches Patentamt

European Patent Office

Office européen des brevets

(11) Publication number: **0 229 530**
**A2**

## EUROPEAN PATENT APPLICATION

(21) Application number: 86310208.3

(22) Date of filing: 30.12.86

(51) Int. Cl.⁴: **C12N 1/18** , C12G 1/02 ,
C12C 11/04 , C12P 7/06 ,
//(C12N1/18,C12R1:865)

(30) Priority: 31.12.85 GB 8531925

(43) Date of publication of application:
22.07.87 Bulletin 87/30

(84) Designated Contracting States:
AT BE CH DE ES FR GB GR IT LI LU NL SE

(71) Applicant: **Bass Public Limited Company**
**137 High Street**
**Burton-on-Trent DE14 1JZ(GB)**

(72) Inventor: **Quain, David Edwin**
**22 Raven Road**
**Yoxall Staffordshire, DE13 8PY(GB)**

(74) Representative: **Wallbank, Roger Wickham et al**
**BARKER, BRETTELL & DUNCAN 138 Hagley Road**
**Edgbaston Birmingham, B16 9PW(GB)**

(54) The propagation of yeast, fermentation employing that yeast and products of that fermentation.

(57) It was previously thought that brewers' yeast, Saccharomyces cerevisiae, was incapable of employing mannitol or sorbitol as a source of carbon during propagation in a catabolite derepressive mode. The invention is based on the discovery that many strains of S. cerevisiae can in fact employ mannitol and/or sorbitol in this way. Simple tests reveal those strains that are suitable. Positive action is preferably taken to introduce oxygen during propagation. A yeast extract or other undefined growth medium may need to be present. Yeast propagated in this manner may be used for the fermentation of fermentable carbohydrates in an aqueous medium, and in particular may be used for brewing beer or for making wine.

# THE PROPAGATION OF YEAST, FERMENTATION EMPLOYING THAT YEAST AND PRODUCTS OF THAT FERMENTATION

This invention relates to the propagation of yeast, fermentation employing that yeast and products of that fermentation. The invention is primarily concerned with the propagation of yeast for use in brewing although it can be applied to the propagation of yeast for use in wine production and other processes.

In a conventional brewing process, a selected strain of Saccharomyces cerevisiae is pitched into wort containing fermentable sugars, a source of assimilable nitrogen and oxygen. Prior to growth and fermentation, dissolved oxygen in the wort is rapidly consumed by the yeast and the process thereafter occurs in the absence of oxygen. During the course of this fermentation the yeast grows on the fermentable sugars producing ethanol. Such metabolism has been described as fermentative or anaerobic, catabolite repressed. When this fermentation process is complete, it is normal brewing practice that the yeast is recovered and recycled in subsequent fermentations. Hence, yeast used to pitch a fermentation may also be considered as being a product of a previous fermentation.

It is well known that in the absence of readily fermentable sugars but in the presence of some other sources of carbon such as ethanol and in the presence of oxygen, S. cerevisiae can propagate in an oxidative or catabolite derepressive mode, rather than a catabolite repressive or fermentative mode. Yeasts propagated in the anaerobic, fermentative mode have little mitochondrial function and contain a low level of sterols. Yeasts propagated in the catabolite derepressive mode, however, have fully-functional mitochondria and accumulate large amounts of sterols. As is well known S. cerevisiae can grow on many different carbon sources which exert varying degrees of catabolite repression. However it has also been reported in a number of published papers that S. cerevisiae cannot be propagated using certain other sources of carbon, such as mannitol and sorbitol.

The present invention is based first on the discovery that those previous reports do not apply to all strains of S. cerevisiae, and secondly that yeasts propagated by methods employing those sources of carbon are of value and can be used to advantage in fermentation processes.

From a first aspect, therefore, the present invention consists in a method of propagating a strain of Saccharomyces cerevisiae in which method the strain is a selected strain and in which the yeast is propagated in a catabolite derepressive mode in the presence of a substrate affording as a source of carbon at least one of the compounds mannitol and sorbitol.

It is thought likely that the work in this field previously reported was carried with strains of S. cerevisiae unable to grow on mannitol and that the results of experiments with those strains were generalized to include all strains of S. cerevisiae. In fact it has now been found that of forty polyploid strains of S. cerevisiae that have so far been examined, twenty can be grown on mannitol and four on sorbitol. Many of these strains were obtained from the National Collection of Yeast Cultures (NCYC). They include NCYC 430 and NCYC 1415, both of which can grow on mannitol and on sorbitol, NCYC 177, NCYC 240 and NCYC 1048 which can grow on mannitol but not on sorbitol. Of six genetically defined haploid strains of S. cerevisiae that were tested, one strain (S. cerevisiae A184D) was found to be capable of growing on mannitol, and none was found to be capable of growing on sorbitol. It is therefore apparent that the selection of a strain of S. cerevisiae suitable for use in carrying out a method in accordance with the first aspect of the present invention is a matter of simple experiment.

Therefore, from a second aspect the present invention consists in a method of propagating a strain of Saccharomyces cerevisiae in which method strains of S. cerevisiae are tested to determine which of them can be propagated in a catabolite derepressive mode in the presence of a substance affording as a source of carbon at least one of the compounds mannitol and sorbitol, selecting from those strains a strain that can be so propagated and propagating it by a method in accordance with the first aspect of the present invention.

In order to encourage propagation to occur in a catabolite derepressive mode it is generally necessary to arrange for propagation to take place in an aqueous liquid medium and for positive action to be taken to introduce oxygen into that medium during propagation. Oxygen may be introduced into the liquid medium by shaking the liquid in the presence of oxygen, air or some other suitable gaseous mixture containing oxygen, or it may be introduced by causing bubbles of such a gaseous substance to be passed through the liquid medium.

The substrate preferably contains no rapidly fermentable sugar or only a very small amounts of such a sugar or such sugars. Nevertheless the presence of a very small amount of a readily fermentable sugar such as glucose may be advantageous in providing an energy source for metabolism.There may for example be about 0.1 g glucose per 100 ml.

It is known that to enable a micro-organism such as a yeast to be propagated it is necessary to include in the substrate a growth medium which normally includes a source of nitrogen, trace metals and possibly buffering salts. Growth media may be categorized as defined and undefined. Defined media are those of which the entire constitution is known in detail. Such media may be made by taking the individual constituents and forming them into an aqueous solution or suspension. Undefined media, on the other hand, are those of which the entire constitution is not known in detail. Such media may contain biological residues which are known to contain certain specific chemical compounds and also to contain substances which are known to fall within certain categories of compounds but the exact nature of which may not be known.Thus, for example, an undefined medium may include a yeast extract known to contain vitamins and nitrogenous material, even though the full details of the constituents are unknown.

Now it has been found that in carrying out a method in accordance with the first aspect of the present invention it is almost always necessary to incorporate an undefined growth medium in the substrate. In particular it has been found that a growth medium including a yeast extract is normally effective. It is believed likely that vitamins present in the extract may well be responsible for rendering the medium effective. Whether or not this is so, there are certainly one or more constituents in undefined growth media of the kind customarily used in growing micro-organisms that appear to be efficacious in enabling or assisting S. cerevisiae to propagate in the manner referred to in the first aspect of the present invention. This being so, if and when that constituent or each of those constituents is identified, it could be specifically included in a defined growth medium which would then be effective. At this time, therefore, the use of an undefined medium appears to be essential, but at some future date it may become possible to provide a defined medium that will be satisfactory.

It is mentioned above that it may be desirable to include in the substrate a small amount of readily fermentable sugar. An undefined growth medium, such as one containing yeast extract, may itself contain enough readily fermentable sugar for the purpose mentioned, so that the addition of readily fermentable sugar from another source then becomes unnecessary.

From a third aspect the present invention consists in a selected strain of S. cerevisiae that has been propagated by a method in accordance with the first aspect or the second aspect of the present invention. S. cerevisiae in accordance with this third aspect of the present invention will hereinafter be referred to for convenience as modified yeast. Likewise, S. cerevisiae that has been propagated in a conventional fermentative mode will hereinafter be referred to as unmodified yeast.

From a fourth aspect the present invention consists in a method of fermentation in which modified yeast (as defined above) is introduced into an aqueous liquid containing fermentable carbohydrates, and fermentation such as to yield ethanol takes place. For convenience such a method will hereinafter be referred to as a modified method of fermentation.

From a fifth aspect the present invention consists in a method of fermentation in which modified yeast is introduced into an aqueous liquid containing fermentable carbohydrates, and fermentation takes place such as to yield an alcoholic beverage, that is a beverage containing ethanol.

This modified method of fermentation is particularly applicable to brewing, that is to brewing beer.In carrying out a brewing process in accordance with the present invention a suitable strain of the modified yeast may be introduced into a wort of a conventional kind and fermentation may be caused or allowed to occur. Such a method of brewing will hereinafter be referred to as a modified method of brewing.

It is to be understood, however, that a modified method of fermentation may also be used for other purposes such as the fermentation of grape juice to produce wine.

It is generally found that in a modified method of fermentation, the rate at which the carbohydrates are converted to ethanol is more rapid than in a method of fermentation which differs from it only in that the yeast employed is an unmodified yeast. In particular, in brewing there is usually a considerable reduction in the time lag that usually occurs between the introduction of the yeast into the wort and the onset of vigorous fermentation. This is thought to result from the presence in modified yeasts of unsaturated fatty acids and sterols that are present only in smaller quantities in similar strains of unmodified yeasts.

Another effect that is generally observed in the course of a modified method of fermentation is that there is a greater increase in the growth of yeast than usually occurs when using an unmodified yeast. For example in a conventional brewing process, employing an unmodified yeast, the yeast cells that have been introduced are able to undergo three rounds of cell division, while in a similar brewing process employing a modified yeast the cell inoculum is able to divide six or more times.

A modified brewing process that is conventional, apart from its use of modified yeast, results in the propagation of the yeast in the fermentative mode. That newly propagated yeast is found to have properties which are the same or substantially the same as those of an unmodified yeast of the same strain. Yeast propagated during a modified brewing process thus lacks the valuable properties of the modified yeast that was used to pitch the wort at the start of the process. It is therefore preferred, when carrying out a modified method of brewing, or any other modified method of fermentation, to use little or no yeast other than modified yeast. Thus, in particular it is preferred not to pitch wort with a mixture of modified yeast and yeast that has been propagated during a previous brewing process.

The yeast that is propagated during a modified method of brewing or other modified method of fermentation may of course be used in other brewing or fermentation processes, but as it resembles an unmodified yeast it is envisaged that most if not all of such yeast would usually be discarded or used for other, non-fermentation purposes such as for the manufacture of yeast extracts, animal foods and the like.

In view of the greatly increased effectiveness of modified yeast it is found that in carrying out a modified method of brewing or other modified method of fermentation the quantity of modified yeast that is required is very much less than the quantity of yeast that would be required in a similar process using unmodified yeast. While a fermentation process using an unreduced quantity of modified yeast may operate reasonably satisfactorily, it is likely to be unnecessarily expensive and also somewhat less efficient than might be expected. It is therefore envisaged that the quantity of modified yeast that is likely to be used in carrying outa modified fermentation process will be very much less than the quantity of unmodified yeast used in a conventional, but otherwise similar, fermentation process. More particularly, in comparison with a conventional brewing process, a modified brewing process may employ a weight of modified yeast that is less than ten per cent of the weight of unmodified yeast.Indeed, that percentage may well be reduced further and be between two and five per cent of the weight of unmodified yeast.

From a sixth aspect the present invention consists in beer or alcoholic other beverage made by a modified method of brewing or other modified method of fermentation (as hereinbefore defined).

The present invention is applicable to the large-scale production of beer and other beverages in a brewery, but in addition it is applicable to the production of such beer and other beverages on a small scale. Such production is often carried out in the home and is often referred to as home brewing.

It is increasingly common for a person wishing to undertake home brewing to acquire an initial small quantity of unmodified yeast, to propagate a stock of yeast from that initial quantity and to use that stock to pitch wort that he or she has prepared. It is envisaged that if a modified yeast were used in place of an unmodified yeast it would always, or at least often, be possible to pitch the wort directly with a small quantity of modified yeast supplied to the person undertaking the brewing, without the intervening step of that person having to propagate the yeast.

Modified yeast for this purpose, or for other purposes, may be stored in the form of a suspension in an aqueous liquid. Thus, from a seventh aspect the present invention consists in a predetermined quantity of modified yeast in a sachet or other closed container, for use in home brewing or home wine-making.

In contrast to unmodified yeast, it is found that modified yeast is stable for prolonged periods in aqueous suspension. A typical suspension may contain from 2 g to 10 g dry weight of yeast per 100 ml. The modified yeast may be suspended in water alone, or other substances may be added to increase the shelf-life of the yeast. For example to increase the shelf-life of modified yeast, mannitol and/or sorbitol may be included; an effective concentration has been found to be 10 g per 100 ml. An antimicrobial agent such as beta-phenol ethanol or a hop isoalpha acid may be included so as to enhance the microbiological stability of the suspension. A quantity of the suspension may be housed in a flexible sachet or sterile bottle.

Examples of the present invention will now be described, by way of example.

Example I

Several strains of S. cerevisiae, known to be suitable for fermentation processes for the production of beer and wine, were tested to discover which of them were capable of being propagated in a catabolite depressive mode.

In each instance, viable cells of the yeast strain concerned were introduced into a 2 l conical flask containing 0.4 l of an aqueous medium containing, per litre,
yeast extract 10 g
bacteriological peptone 5 g
ammonium sulphate 1.2 g

sodium chloride 0.5 g
calcium chloride dihydrate 0.1 g
magnesium chloride heptahydrate 0.7 g
potassium dihydrogen phosphate dihydrate 1 g
ferric chloride 0.003 g
mannitol or sorbitol 50 g

The flasks were shaken continuously at 200 rpm, and the medium was maintained at a temperature of 30°C, for 48 hours. It was found that some of the strains propagated in those conditions and that others did not.Those that did propagate successfully were selected as being suitable for use in carrying out fermentation processes in accordance with the present invention.

It was found in separate tests, for example, that the wine yeast NCYC 430 propagated successfully on mannitol and on sorbitol.

A proprietary ale yeast designated BBI, currently used in a conventional beer-brewing process, was found to be capable of using mannitol but not sorbitol as a source of carbon.

Other readily available yeasts that can be successfully propagated in this manner are also referred to above.

Propagation of a selected yeast, in the manner described, resulted in yields of yeast in excess of 1 gm of modified yeast (dry weight) per 100 ml.

## Example II

A sample of each of the modified yeasts, propagated as described in Example I, was prepared in such a manner as to make it suitable for use in home brewing or home wine-making. To this end, aqueous suspensions of the modified yeasts were prepared. Each suspension comprised 10 g modified yeast (dry weight) per 100 ml of a solution of mannitol or sorbitol in water, containing 10 g mannitol or 10 g sorbitol per 100 l 6 ml of each suspension was sealed into a sachet. After storage for a prolonged period -approximately a year in each instance -the viability of the yeast suspension was substantially undiminished.

## Example III

6 ml of a suspension of a modified proprietary yeast BB1, prepared as described in Example II, was used to pitch five gallons (22.7 l) of a conventional wort of specific gravity 1.040.

Fermentation occurred rapidly and was allowed to continue for a normal period. The resultant beer was treated in the manner customary in home brewing, and after some further sugar and modified yeast had been added, the beer was bottled and allowed to undergo secondary fermentation in the capped bottles. The final product was similar to beer brewed in a similar manner but using much larger quantities of unmodified yeast.

Similar results were obtained when using modified yeast NCYC 240.

## Example IV

2 ml of a suspension of modified yeast NCYC 430, prepared as described in Example II, was used to pitch one gallon (4.55 l) of a conventional grape-must of specific gravity 1.090. After fermentation at room temperature, for a normal period, the resultant wine was treated in the manner customary in the home brewing of wine. The final product was to be superior to wine produced in a similar manner but using a much larger quantity of unmodified active NCYC 430 yeast.

It has been found that usually, if not always, a modified yeast is cohesive. In consequence it tends to accumulate at the bottom of any vessel in which fermentation occurs. Thus during a primary fermentation the yeast tends to form a sediment at the bottom of the fermentation vessel, and during secondary fermentation it tends to form a sediment at the bottom of the bottle. In each instance this property of the yeast causes the supernatant liquid to be substantially free of yeast, with the result that it can be readily decanted.

It will be appreciated from the foregoing that modified yeasts have a number of advantages as compared with unmodified yeasts. In addition to being cohesive, they enable beer and wine fermentations to be effected with much reduced quantities of yeast and they can readily be stored for prolonged periods.

## Claims

1. A method of propagating a strain of Saccharomyces cerevisiae characterised in that the strain is a selected strain and in which the yeast is propagated in a catabolite derepressive mode in the presence of a substrate affording as a source of carbon at least one of the compounds mannitol and sorbitol.

2. A method of propagating a strain of Saccharomyces cerevisiae characterised in that strains of S. cerevisiae are tested to determine which of them can be propagated in a catabolite derepressive mode in the presence of a substance affording as a source of carbon at least one of the compounds mannitol and sorbitol, selecting from those strains a strain that can be so propagated and propagating it by a method according to claim 1.

3. A method according to either of claims 1 and 2 characterised in that propagation takes place in an aqueous liquid medium and positive action is taken to introduce oxygen into that medium during propagation.

4. A method according to any one of the preceding claims characterised in that the propagation takes place in the presence of a very small proportion of a readily fermentable sugar which provides an energy source for metabolism.

5. A method according to claim 4 characterised in that there is about 0.1 g readily fermentable sugar per 100 ml of aqueous liquid medium in which propagation takes place.

6. A method according to any one of the preceding claims characterised in that propagation takes place in the presence of an undefined growth medium.

7. A method according to claim 6 characterised in that the undefined growth medium comprises a yeast extract.

8. A selected strain of S. cerevisiae characterised in that it has been propagated by a method in accordance with any one of the preceding claims.

9. A method of fermentation characterised in that yeast propagated by a method in accordance with any one of claims 1 to 7 is introduced into an aqueous liquid containing fermentable carbohydrates, and fermentation such as to yield ethanol takes place.

10. A method of fermentation characterised in that yeast propagated by a method in accordance with any one of claims 1 to 7 is introduced into an aqueous liquid containing fermentable carbohydrates, and fermentation takes place such as to yield an alcoholic beverage.

11. A method according to claim 10 in which the beverage is beer.

12. A method according to claim 10 in which the beverage is a wine.

13. An alcoholic beverage made characterised in that it is made by a method in accordance with any one of claims 10 to 12.

14. For use in home brewing or home winemaking, a sachet or other closed container characterised in that it contains an aqueous suspension of a predetermined quantity of yeast propagated by a method in accordance with any one of claims 1 to 7.

15. A sachet or other container according to claim 14, in which the aqueous suspension also includes at least one of the compounds mannitol and sorbitol to increase the shelf-life of the yeast.